# FASCICULE DE BREVET EUROPEEN

(11) **EP 2 558 131 B1**
(45) Date de publication et mention de la délivrance du brevet: **22.02.2017**
(21) Numéro de dépôt: 11730383.4
(22) Date de dépôt: 15.04.2011
(51) Int. Cl.: A61L 9/12

(54) **DIFFUSEUR DE FRAGRANCE A RETOURNEMENT**
DUFTSTOFF-UMKEHRZERSTÄUBER
REVERSE FRAGRANCE DIFFUSER

(30) Priorité: 16.04.2010 FR 1052901
(43) Date de publication de la demande: 20.02.2013
(73) Titulaire: Diptyque, 75005 Paris (FR)
(72) Inventeur: BOUET, Loïc, F-76100 Rouen (FR); LEQUERRE, Frédéric, F-27190 Nogent Le Sec (FR)
(74) Mandataire: Laget, Jean-Loup
(86) Numéro de dépôt international: PCT/FR2011/050873
(87) Numéro de publication internationale: WO 2011/128604

(56) Documents cités:
- EP-A1- 0 864 330
- EP-A1- 1 088 562
- WO-A2-2005/032606
- US-A1- 2007 023 541

## Description

La présente invention se rapporte à un diffuseur de fragrance destiné à répandre un parfum ou une odeur dans un espace.

Parmi les dispositifs utilisés pour répandre un parfum dans une pièce, il est connu d'utiliser un atomiseur de type aérosol pour vaporiser une solution parfumée. Dans ce cas, le produit est diffusé par l'aérosol sous forme de particules de dimensions importantes. Cette solution implique la présence d'alcool et d'autres solvants dans la solution parfumée, et d'un point de vue pratique, elle donne lieu à une diffusion trop rapide des substances emmagasinées. Le document EP 0 864 330 décrit un diffuseur de parfum à deux compartiments communiquant par un orifice, et à deux tubes axiaux ouverts sur l'extérieur pour la diffusion du parfum.

On connaît également par les documents EP 1 088 562 et US 2007/0023 541, des dispositifs comportant un réservoir renfermant un liquide parfumant, et une mèche comportant une partie baignant dans le liquide et une partie émergée qui est au contact de l'air environnant. Le liquide est absorbé par la mèche par capillarité, et il s'évapore via la partie émergée pour se répandre dans l'espace environnant. Avec cette solution, l'évaporation se déroule continuellement, de sorte qu'elle est difficilement dosable. D'autre part, un utilisateur peut être au contact du liquide parfumant, ce qui limite la composition de ce liquide.

Le document WO 2005/032606 décrit un diffuseur de parfum constitué de deux flacons réunis par une mèche partiellement à l'air libre et qui assure la diffusion du parfum.

Il est également connu d'utiliser comme liquide parfumant une huile essentielle, c'est à dire un extrait de parfum mélangé à de l'huile. L'huile essentielle est alors placée dans un réceptacle chauffé par un dispositif approprié pour répandre le parfum qu'elle renferme par évaporation. Cette autre solution implique elle aussi la présence d'alcool dans le liquide parfumant, et elle nécessite d'autre part que le dispositif utilisé soit chauffant. De plus, le récipient doit être nettoyé avant de mettre une nouvelle huile lorsqu'on souhaite changer de parfum.

Selon une autre solution décrite dans la demande de brevet FR 2 774 597, la diffusion est assurée avec un dispositif comportant une base portant différents modules. Chaque module, qui comporte une enceinte ajourée renfermant des granulés imbibés de parfum, peut être soit ouvert soit fermé. La base est équipée d'un ventilateur pour établir un flux d'air traversant chaque module ouvert afin de répandre un parfum dans l'environnement. Cependant, l'enceinte ajourée comporte des orifices qui doivent être conçus pour ne pas être bouchés par les granulés que renferme l'enceinte, ce qui rend ce réservoir coûteux. En pratique, l'établissement d'un flux d'air traversant de façon appropriée les granulés entassés dans l'enceinte n'est pas simple à établir. En pratique, un brassage satisfaisant du parfum et de l'air ventilé ne peut finalement pas être assuré dans des conditions satisfaisantes.

La présente invention a pour but de proposer un nouveau diffuseur de fragrance qui évite tout ou partie des inconvénients précités et notamment un diffuseur de fragrance permettant d'interrompre brutalement la diffusion et ce de façon simple et rapide pour l'utilisateur, tout en étant esthétique et peu onéreux.

A cet effet, l'invention a pour objet un diffuseur de fragrance comportant un premier flacon pouvant comprendre la fragrance, et monté sur ce premier flacon, un système de diffusion sur lequel est monté un deuxième flacon, caractérisé en ce que ledit système de diffusion comporte :
- deux bouchons-verseurs montés tête-bêche et présentant chacun un tube d'écoulement,
- une plaquette de matière poreuse montée entre les deux bouchons-verseurs, et
- une bague extérieure pourvue, en regard de la plaquette en matière poreuse, d'au moins un orifice de diffusion de la fragrance,
chacun desdits bouchons-verseurs comprenant en outre au moins un trou d'écoulement permettant le passage de la fragrance du flacon vers la plaquette de matière poreuse.

De par les caractéristiques susmentionnées, le diffuseur de fragrance présente l'avantage d'être pivotable lors de son utilisation notamment lorsqu'il est relié à un deuxième flacon. Ainsi, la fragrance contenue dans le premier flacon passera grâce au système de diffusion vers le second flacon, la fragrance étant diffusée via la plaquette poreuse vers l'orifice de diffusion de la bague. Après transvasement, l'utilisateur aura simplement à retourner le diffuseur pour que la fragrance passe désormais du deuxième flacon vers le premier afin d'être diffusée et ceci jusqu'à évaporation de ladite fragrance. Ainsi, l'utilisateur peut retourner et donc actionner le diffuseur lorsqu'il le souhaite ou en a besoin. En l'absence de deuxième flacon, le diffuseur est à usage unique. Dans une alternative, le deuxième flacon peut être remplacé par un bouchon. Ceci présente notamment un avantage pour le conditionnement du diffuseur.

De préférence, le tube d'écoulement de chaque bouchon-verseur traverse l'autre bouchon-verseur après avoir traversé ladite plaquette en matière poreuse.

Avantageusement, chaque bouchon-verseur présente pour le tube d'écoulement de l'autre bouchon-verseur, une ouverture de diamètre supérieur à celui dudit tube d'écoulement.

Préférentiellement, chaque bouchon-verseur présente sur sa face en regard de ladite plaquette en matière poreuse une surface en entonnoir autour de ladite ouverture.

De manière avantageuse, les deux bouchons-verseurs sont identiques.

En particulier, l'orifice de diffusion de la fragrance porté par la bague extérieure est à ouverture réglable.

De préférence, la plaquette en matière poreuse est en mousse en polyéthylène, en feutre, en fibres naturelles ou en un de leurs mélanges.

La présente invention a également pour objet l'utilisation d'un diffuseur de fragrance selon l'une des caractéristiques susmentionnées, pour former un diffuseur de fragrance apte à être retourné, de sorte que la fragrance passe du premier flacon vers le deuxième, puis du deuxième jusqu'au premier par le tube d'écoulement et par le trou d'écoulement.

De préférence, le diffuseur de fragrance est utilisé pour parfumer une pièce.

L'invention sera mieux comprise, et d'autres buts, détails, caractéristiques et avantages de celle-ci apparaîtront plus clairement au cours de la description suivante d'un mode de réalisation particulier de l'invention, donné uniquement à titre illustratif et non limitatif, en référence aux dessins annexés.

Sur ces dessins :
- la figure 1 représente une vue en coupe longitudinale du diffuseur de fragrance selon un mode de réalisation de la présente invention en mode de fonctionnement ;
- la figure 2 représente une vue en coupe longitudinale et agrandie du système de diffusion du diffuseur de la figure 1.

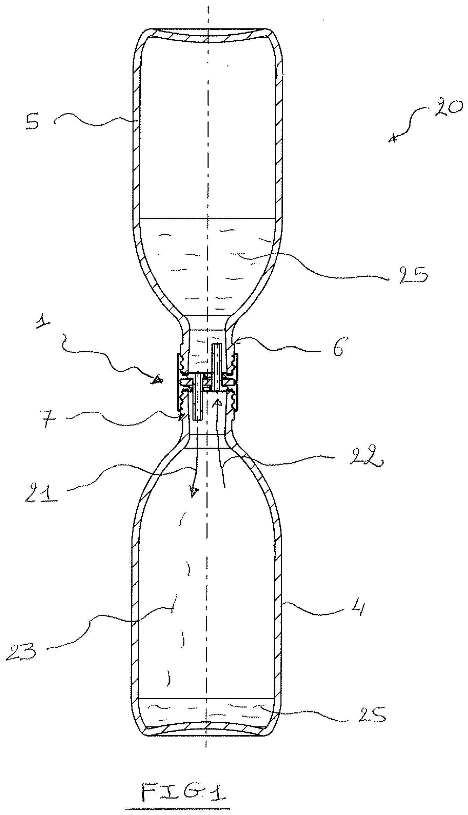

Tel que représenté sur la figure 1, un diffuseur de fragrance 20 selon l'invention est représenté et comporte un premier flacon 5, un deuxième flacon 4 et entre les deux, un système de diffusion 1.

Chaque flacon 4 et 5 comprend un bec verseur (7 et 6 respectivement). Les flacons 4, 5 rejoignent d'ailleurs le système de diffusion 1 au niveau de leur goulot 6 ou 7. Ces deux goulots pourront être séparés d'une hauteur de l'ordre de 8 à 10 mm. Comme le montre la figure 1, le premier flacon 5 contient une fragrance 25 et est disposé au dessus, tandis que le deuxième flacon 4 est disposé au dessous et contient de l'air. La fragrance du premier flacon va passer par simple gravité vers le système de diffusion 1, puis vers le deuxième flacon (flèche 21 et gouttes de fragrance 23), tandis que de l'air du deuxième flacon 4 va faire le chemin inverse (flèche 22) et passer du flacon 4 vers le premier flacon 5 sous l'effet de la dépression liée à l'écoulement de la fragrance et afin de compenser la perte en fragrance du flacon 5.

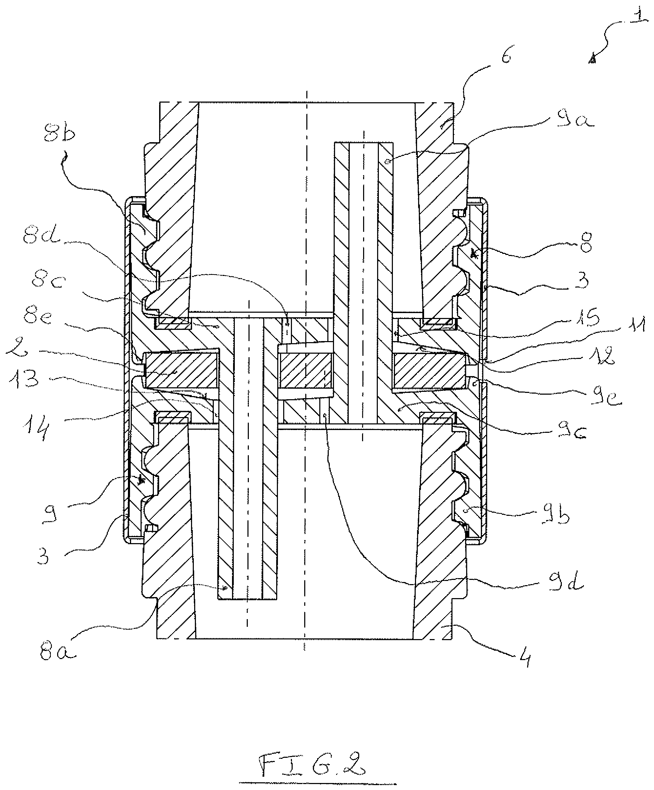

Le système de diffusion va être décrit plus amplement ci-dessous en se référant à la figure 2.

Ce système de diffusion 1 comprend quatre éléments principaux, à savoir deux bouchons-verseurs 8 et 9 présentant chacun un tube d'écoulement 8a et 9a, une plaquette de matière poreuse 2 disposée entre les deux bouchons-verseurs 8, 9, et une bague extérieure 3 enveloppant à la fois les bouchons-verseurs 8 et 9 et la plaquette en matière poreuse 2, hormis à un endroit correspondant à un orifice de diffusion 11 de la fragrance.

Les bouchons-verseurs 8 et 9 sont identiques. Ceci présente un avantage certain en ce qui concerne les coûts de fabrication qui sont réduits du fait qu'il suffit de ne produire qu'un moule unique et qu'une pièce en série. La fabrication se trouve en outre facilitée.

Chaque bouchon-verseur 8, 9 présente une section sensiblement en U, c'est-à-dire deux parois latérales, respectivement 8b, 9b, sensiblement parallèles reliées par une base transversale, respectivement 8c, 9c.

Sur cette base transversale 8c ou 9c sont agencés un tube d'écoulement 8a ou 9a du bouchon-verseur 8 ou 9, ainsi que des trous d'écoulement 8d ou 9d ayant pour fonction de laisser passer la fragrance du flacon 5 vers la plaquette en matière poreuse 2, ou du flacon 4 vers la plaquette en matière poreuse 2, selon que le flacon 5 ou 4 se trouve en dessus. Ces trous d'écoulement 8d ou 9d présentent par exemple un diamètre de 0,8 mm. Ainsi, pendant la phase d'écoulement, la fragrance qui est liquide, va imprégner par ces trous d'écoulement 8d et 9d, la plaquette en matière poreuse.

La plaquette en matière poreuse 2 peut-être en mousse comme de la mousse de polyéthylène (porex®), en fibres naturelles, en feutre ou un de leurs mélanges. Elle pourra présenter une épaisseur de 3 mm environ et présentera classiquement une forme en disque.

Les tubes d'écoulement 8a et 9a des bouchons-verseurs 8 et 9 sont excentrés et montés tête-bêche. Pour cela, la base horizontale 8c du bouchon-verseur 8 comprend une ouverture 15 apte à laisser passer le tube d'écoulement 9a du bouchon-verseur 9 et la base horizontale 9c du bouchon-verseur 9 comprend une ouverture 14 apte à laisser passer le tube d'écoulement 8a du bouchon-verseur 8. De même, la plaquette de matière poreuse 2 disposée entre les deux bouchons-verseurs 8, 9 comprend des orifices 3 afin de pouvoir être traversée par les tubes d'écoulement 8a et 9a. En particulier, le diamètre des ouvertures 14 et 15 est supérieur au diamètre extérieur du tube d'écoulement 8a ou 9a, de sorte à former un passage d'écoulement autour des tubes d'écoulement 8a ou 9a. Ainsi, lorsque la plaquette de matière poreuse sera saturée de fragrance, le superflu de fragrance s'écoulera vers ces passages d'écoulement. Les tubes d'écoulement 8a, 9a présentent par exemple une hauteur d'environ 20 à 25 mm, telle que 24 mm et un diamètre interne de l'ordre 2,5 mm.

Dans le même but de rediriger le superflu de fragrance, la face des bases 8c et 9c des bouchons-verseurs 8 et 9, qui est disposée en regard de la plaquette en matière poreuse 2, comprend respectivement un entonnoir 12 ou 13 autour de leur ouverture 15 ou 14. Cette forme en entonnoir fait ainsi office de collecteur afin que le surplus de fragrance ne coule pas vers l'extérieur du système de diffusion 1 mais soit reguidé vers l'intérieur du contenant du bas, ici le flacon 4 d'après la figure 1.

Comme indiqué plus haut, la plaquette de matière poreuse 2 est disposée entre les deux bouchons-verseurs 8 et 9. Elle est en particulier maintenue en place par une butée 8e, 9e disposée à une des extrémités de la paroi latérale 8b ou 9b respectivement, c'est à dire celle la plus proche de la plaquette 2. Les deux butées 8e et 9e sont séparées d'une faible distance, par exemple environ 0,5 à 1 mm, de sorte à former une ouverture pour permettre la diffusion de la fragrance vers l'extérieur.

Chaque paroi latérale 8b ou 9b du bouchon-verseur 8 ou 9 comporte également le long de sa surface intérieure des moyens d'attache à un bouchon ou à un flacon, le bouchon ou le flacon étant généralement vissé sur ces moyens d'attache. Ceux-ci pourront présenter une hauteur de 13 à 14 mm par exemple.

Enfin, le système de diffusion comporte une bague extérieure 3. Cette bague 3 enveloppe les parois latérales 8b et 9b des bouchons-verseurs 8 et 9 et comporte un orifice de diffusion 11, voire plusieurs. Cet orifice de diffusion 11 pourra par exemple avoir un diamètre de l'ordre de 1,5 mm à 2 mm et sera de préférence placé en regard de la plaquette en matière poreuse 2 et en particulier en regard de l'ouverture formée par les deux butées 8e et 9e. Cette bague 3 pourra être réglée par exemple par pivotement par l'utilisateur. Ainsi, si celui-ci désire une diffusion de la fragrance, il suffira de mettre l'orifice de diffusion 11 en regard de l'ouverture formée par les butées 8e et 9e et s'il désire stopper le système de diffusion 1, la bague extérieure 3 comprendra un moyen (non représenté) permettant l'occlusion de l'orifice de diffusion 11.

Le temps découlement de la fragrance d'un flacon à l'autre pourra en outre être réglé en fonction des diamètres des trous d'écoulement 8d, 9d, et des tubes d'écoulement 8a et 9a et de la viscosité de la fragrance.

De même, en fonction du volume de la pièce à parfumer, l'épaisseur de la plaquette en matière poreuse 2, et la section de l'orifice de diffusion 11 sont ajustables.

Le fonctionnement du diffuseur va être décrit brièvement ci-dessous en se référant aux figures 1 et 2.

Tout d'abord, lors du conditionnement du diffuseur 20, les flacons 4 et 5 ne seront pas forcément connectés l'un à l'autre via le système de diffusion 1. En effet, l'un des tubes 4, 5, par exemple le tube 5 contenant la fragrance pourra être bouché par vissage avec un bouchon de sorte que la fragrance 25 n'imbibe déjà pas la plaquette en matière poreuse 2. Dans ce cas, le système de diffusion 1 sera monté sur le flacon 4 vide. Il suffira ainsi pour l'utilisateur de dévisser le bouchon du flacon 5 et de visser ce flacon contenant la fragrance 25 sur le système de diffusion 1.

Ensuite, il suffira à l'utilisateur de placer le flacon 5 en dessus du flacon 4, afin que la fragrance s'écoule en goutte à goutte et imbibe la plaquette poreuse 2 via le trou d'écoulement 8d. Une fois la plaquette poreuse 2 imbibée, la fragrance 25 va se diffuser à travers l'ouverture formée par les butées 8e et 9e, et l'orifice de diffusion 11 et cela jusqu'à ce que toute la fragrance 25 contenue dans la plaquette poreuse 2 se soit évaporée.

Il suffira alors à l'utilisateur, pour avoir de nouveau une diffusion d'odeur, de retourner le diffuseur 20 afin que le flacon 4, qui contient désormais la fragrance 25, soit disposé au dessus. De même, la fragrance 25 va couler et imbiber la plaquette poreuse 2 via le trou d'écoulement 9d. Une fois la plaquette poreuse 2 imbibée, la fragrance 25 va se diffuser à travers l'ouverture formée par les butées 8e et 9e, et l'orifice de diffusion 11 et cela jusqu'à ce que toute la fragrance 25 contenue dans la plaquette poreuse 2 se soit évaporée.

L'utilisateur pourra retourner le diffuseur jusqu'à diffusion de la totalité de la fragrance.

En outre, comme indiqué plus haut, s'il ne souhaite pas de diffusion d'odeur, il suffit de fermer l'orifice de diffusion 11 par un moyen prévu sur la bague extérieure 3.

Bien que l'invention ait été décrite en liaison avec un mode de réalisation particulier, il est bien évident qu'elle n'y est nullement limitée et qu'elle comprend tous les équivalents techniques des moyens décrits ainsi que leurs combinaisons si celles-ci entrent dans le cadre de l'invention.

## Revendications

1. Diffuseur (20) de fragrance (25) comportant un premier flacon (5) pouvant comprendre la fragrance, et monté sur ce premier flacon (5), un système de diffusion (1) sur lequel est monté un deuxième flacon (4), **caractérisé en ce que** ledit système de diffusion (I) comporte :
- deux bouchons-verseurs (8, 9) montés tête-bêche et présentant chacun un tube d'écoulement (8a, 9a),
- une plaquette de matière poreuse (2) montée entre les deux bouchons-verseurs (8, 9), et
- une bague extérieure (3) pourvue, en regard de la plaquette en matière poreuse (2), d'au moins un orifice de diffusion (11) de la fragrance (25),
chacun desdits bouchons-verseurs (8, 9) comprenant en outre au moins un trou d'écoulement (8d, 9d) permettant le passage de la fragrance (25) du flacon (4, 5) vers la plaquette de matière poreuse (2).

2. Diffuseur de fragrance selon la revendication 1, dans lequel le tube d'écoulement (8a, 9a) de chaque bouchon-verseur (8, 9) traverse l'autre bouchon-verseur (9, 8) après avoir traversé ladite plaquette en matière poreuse (2).

3. Diffuseur de fragrance selon la revendication 2, dans lequel chaque bouchon-verseur (8, 9) présente pour le tube d'écoulement (9a, 8a) de l'autre bouchon-verseur, une ouverture (15, 14) de diamètre supérieur à celui dudit tube d'écoulement.

4. Diffuseur de fragrance selon la revendication 3, dans lequel chaque bouchon-verseur (8, 9) présente sur sa face en regard de ladite plaquette en matière poreuse (2) une surface en entonnoir (12, 13) autour de ladite ouverture (15, 14).

5. Diffuseur de fragrance selon l'une des revendications précédentes, dans lequel les deux bouchons-verseurs (8, 9) sont identiques.

6. Diffuseur de fragrance selon l'une des revendications 1 à 5, dans lequel l'orifice de diffusion (11) de la fragrance (25) porté par la bague extérieure (3) est à ouverture réglable.

7. Diffuseur de fragrance selon l'une des revendications précédentes, dans lequel la plaquette en matière poreuse (2) est en mousse en polyéthylène, en feutre, en fibres naturelles ou en un de leurs mélanges.

8. Utilisation d'un diffuseur (20) de fragrance selon l'une des revendications 1 à 7, pour former, un diffuseur de fragrance apte à être retourné, de sorte que la fragrance passe du premier flacon (5) vers le deuxième, puis du deuxième jusqu'au premier par le tube d'écoulement (8a, 9a) et par le trou d'écoulement (8d, 9d).

9. Utilisation d'un diffuseur (20) de fragrance selon la revendication 8, pour parfumer une pièce.

## Patentansprüche

1. Zerstäuber (20) von Duftstoff (25), aufweisend eine erste Flasche (5), die den Duftstoff enthalten kann, und montiert auf dieser ersten Flasche (5), ein Zerstäubersystem (1), auf welchem eine zweite Flasche (4) montiert ist, **dadurch gekennzeichnet, dass** das Zerstäubersystem (1) aufweist:
- zwei Kopf-an-Fuß montierte Gießstopfen (8, 9), die jeweils ein Gießrohr (8a, 9a) aufweisen,
- eine Platte aus porösem Material (2), die zwischen den zwei Gießstopfen (8, 9) montiert ist, und
- einen Außenring (3), der gegenüber der Platte aus porösem Material (2) mit mindestens einer Zerstäuberöffnung (11) des Duftstoffs (25) ausgestattet ist,
wobei jeder der Gießstopfen (8, 9) ferner mindestens ein Gießrohr (8d, 9d) umfasst, das den Durchgang des Duftstoffs (25) aus der Flasche (4, 5) zur Platte aus porösem Material (2) erlaubt.

2. Duftstoff-Zerstäuber nach Anspruch 1, wobei das Gießrohr (8a, 9a) jedes Gießstopfens (8, 9) den anderen Gießstopfen (9, 8) durchquert, nachdem es die Platte aus porösem Material (2) durchquert hat.

3. Duftstoff-Zerstäuber nach Anspruch 2, wobei jeder Gießstopfen (8, 9) für das Gießrohr (9a, 8a) des anderen Gießstopfens eine Öffnung (15, 14) mit einem Durchmesser aufweist, der größer als der Durchmesser des Gießrohrs ist.

4. Duftstoff-Zerstäuber nach Anspruch 3, wobei jeder Gießstopfen (8, 9) auf seiner Fläche gegenüber der Platte aus porösem Material (2) eine trichterförmige Oberfläche (12, 13) um die Öffnung (15, 14) aufweist.

5. Duftstoff-Zerstäuber nach einem der vorangehenden Ansprüche, wobei die zwei Gießstopfen (8, 9) identisch sind.

6. Duftstoff-Zerstäuber nach einem der Ansprüche 1 bis 5, wobei die vom Außenring (3) getragene Zerstäuberöffnung (11) des Duftstoffs (25) eine einstellbare Öffnung hat.

7. Duftstoff-Zerstäuber nach einem der vorangehenden Ansprüche, wobei die Platte aus porösem Material (2) aus Polyethylen-Schaumstoff, aus Filz, aus Naturfasern oder aus einem ihrer Gemische ist.

8. Verwendung eines Duftstoff-Zerstäubers (20) nach einem der Ansprüche 1 bis 7 zur Bildung eines Duftstoff-Zerstäubers, der imstande ist, umgedreht zu sein, damit der Duftstoff aus der ersten Flasche (5) in die zweite gelangt, danach aus der zweiten in die erste durch das Gießrohr (8a, 9a) und durch das Gießrohr (8d, 9d).

9. Verwendung eines Duftstoff-Zerstäubers (20) nach Anspruch 8 zum Parfümieren eines Raums.

## Claims

1. A fragrance (25) diffuser (20) including a first bottle (5) able to comprise the fragrance, and mounted on this first bottle (5), a diffusion system (1) on which a second bottle (4) is mounted, **characterized in that** said diffusion system (1) includes:
- two pourers (8, 9) mounted head to tail and each having a flow tube (8a, 9a),
- a pad of porous material (2) mounted between the two pourers (8, 9), and
- an outer ring (3) provided, across from the pad of porous material (2), with at least one diffusion orifice (11) for the fragrance (25),
each of the pourers (8, 9) further comprising at least one flow hole (8d, 9d) allowing the fragrance (25) to pass from the bottle (4, 5) toward the pad of porous material (2).

2. The fragrance diffuser according to claim 1, wherein the flow tube (8a, 9a) of each pourer (8, 9) traverses the other pourer (9, 8) after having traversed said pad of porous material (2).

3. The fragrance diffuser according to claim 2, wherein each pourer (8, 9) has, for the flow tube (9a, 8a) of the other pourer, an opening (15, 14) having a diameter larger than that of said flow tube.

4. The fragrance diffuser according to claim 3, wherein each pourer (8, 9) has, on its face opposite said pad of porous material (2), a funnel surface (12, 13) around said opening (15, 14).

5. The fragrance diffuser according to one of the preceding claims, wherein the two pourers (8, 9) are identical.

6. The fragrance diffuser according to one of claims 1 to 5, wherein the diffusion orifice (11) for the fragrance (25) supported by the outer ring (3) has an adjustable opening.

7. The fragrance diffuser according to one of the preceding claims, wherein the pad of porous material (2) is made from polyethylene foam, felt, natural fibers or a mixture thereof.

8. A use of a fragrance diffuser (20) according to one of claims 1 to 7, to form a fragrance diffuser able to be turned over, such that the fragrance goes from the first bottle (5) toward the second, then from the second to the first via the flow tube (8a, 9a) and via the flow hole (8d, 9d).

9. The use of a fragrance diffuser (20) according to claim 8, to scent a room.
